(19) Europäisches Patentamt
European Patent Office
Office européen des brevets

(11) Veröffentlichungsnummer : **0 107 163**
**B1**

(12) **EUROPÄISCHE PATENTSCHRIFT**

(45) Veröffentlichungstag der Patentschrift : **30.07.86**

(51) Int. Cl.[4] : **C 07 F 9/18**, C 07 F 9/24, C 07 F 9/12, A 01 N 57/14, A 01 N 57/30

(21) Anmeldenummer : **83110328.8**

(22) Anmeldetag : **17.10.83**

(54) **Dihalogenvinylphenyl-phosphorsäureester, Verfahren zu ihrer Herstellung und ihre Verwendung als Schädlingsbekämpfungsmittel.**

(30) Priorität : **23.10.82 DE 3239288**

(43) Veröffentlichungstag der Anmeldung : **02.05.84 Patentblatt 84/18**

(45) Bekanntmachung des Hinweises auf die Patenterteilung : **30.07.86 Patentblatt 86/31**

(84) Benannte Vertragsstaaten : **AT BE CH DE FR GB IT LI NL**

(56) Entgegenhaltungen :
**DE-A- 2 411 809**
**FR-A- 2 205 526**
**DERWENT JAPANESE PATENTS, Band 7, Nr. 47, Woche vom 21.11.68-27.11.68, Teil 5, Seite 7**

(73) Patentinhaber : **BASF Aktiengesellschaft**
**Carl-Bosch-Strasse 38**
**D-6700 Ludwigshafen (DE)**

(72) Erfinder : **Schwarz, Gerd-Ulrich, Dr.**
**Keltenstrasse 13**
**D-6707 Schifferstadt (DE)**
Erfinder : **Adolphi, Heinrich, Dr.**
**Kalmitweg 11**
**D-6703 Limburgerhof (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft Dihalogenvinylphenylphosphorsäureester, ein Verfahren zu ihrer Herstellung und Schädlingsbekämpfungsmittel, die diese Verbindungen als Wirkstoffe enthalten.

Alkylvinylphenylphosphorsäureester sind als insektizid wirkende Substanzen bekannt (DE-A-2 411 809), ebenso halogenierte Phenylvinyl-phosphate (DE-A-1 567 092).

Außerdem sind aus der FR-A-2 205 526 Allyl- bzw. Allyloxyphenyl-phosphorsäuester bekannt, die im Allylrest z. B. chlorsubstituiert sind, und aus der JP-A-6 827 358 kennt man Vinylphenyl-phosphorsäureester, deren Vinylrest durch Cyan disubstituiert ist.

Verbindungen der vorgenannten Art haben nur theoretisches Interesse gefunden und sind gegenwärtig nicht als Handelsprodukte bekannt ; sie scheinen auch nicht leicht zugänglich zu sein.

Es wurde gefunden, daß Dihalogenvinylphenyl-phosphorsäureester der Formel I

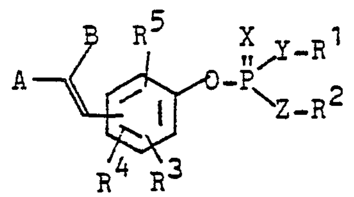

(I)

in der

A, B unabhängig voneinander Fluor, Chlor, Brom oder Trihalogenmethyl,

X Sauerstoff oder Schwefel

Y, Z unabhängig voneinander Sauerstoff, Schwefel oder den zweiwertigen Rest —NH—

$R^1$ Alkyl mit 1 bis 3 Kohlenstoffatomen,

$R^2$ Alkyl, Halogenalkyl, Alkoxyalkyl, Alkylthioalkyl mit 1 bis 6 Kohlenstoffatomen

$R^3$, $R^4$, $R^5$ unabhängig voneinander Wasserstoff, Halogen, Nitro, Cyano, Alkyl, Alkoxy, Alkoxyalkoxy, Alkylthioalkylthio, Alkoxyalkyl, Alkylthioalkyl, Alkylthio, Trihalogenmethyl oder Trihalogenmethoxy bedeuten, Schädlinge aus der Klasse der Insekten, Spinnentiere und Nematoden wirksam bekämpfen. Sie sind den bekannten Halogenphenylvinylphosphaten in ihrer Wirkung überlegen.

Die Verbindungen der Formel I können auf bekannte Weise hergestellt werden, z. B. durch Umsetzung eines entsprechenden Phosphorsäureesterhalogenids mit einem entsprechend substituierten Phenol.

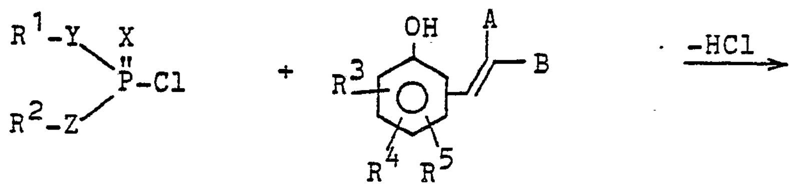


(I)

(II) (III)

Die Umsetzung kann in einem organischen Verdünnungsmittel, wie Acetonitril, Toluol, Methylethylketon, oder in Zwei-Phasen-Systemen, wie Toluol/Wasser, Dichlormethan/Wasser, durchgeführt werden.

Zweckmäßigerweise setzt man die 1- bis 2-molare Menge, bezogen auf Phenol, eines säurebindenden Mittels, d. h. einer Base zu. Vorzugsweise wird ein Überschuß von etwa 10 % verwendet. Geeignet sind anorganische Basen wie Alkalimetallcarbonate, z. B. Kaliumcarbonat, Alkalihydroxide, z. B. Natriumhydroxid, oder tertiäre Amine, z. B. Triethylamin. Aus der Base und dem Phenol kann vorher das Salz hergestellt und dieses mit dem Phosphorsäureesterchlorid umgesetzt werden. Als Salze kommen Alkalimetallsalze, Erdalkalimetallsalze oder gegebenenfalls substituierte Ammoniumsalze, wie Natrium-, Kalium-, Calcium- oder Ammoniumsalze in Betracht.

Eine ausreichende Reaktionsgeschwindigkeit wird i. a. bei einer Temperatur unterhalb von 100 °C, vorzugsweise zwischen 20 und 70 °C bei atmosphärischem Druck erzielt.

Man setzt die Ausgangsstoffe zweckmäßig in stöchiometrischem Verhältnis ein. Ein Überschuß des einen oder anderen Reaktionsteilnehmers kann in einigen Fällen Vorteile bringen.

Das Reaktionsgemisch wird in üblicher Weise aufgearbeitet, z. B. durch Versetzen mit Wasser und Trennen der Phasen. Die Rohprodukte können durch Destillation oder Säulenchromatographie gereinigt werden.

Folgende Verfahren führen ebenfalls zu erfindungsgemäßen Verbindungen :

Dihalogenvinyl-phenyl-thiophosphorsäureester der Formel Ia lassen sich in einer Arbusow-Reaktion durch Umsetzung von Phosphorigsäureestern der Formel IV mit Sulfenylchloriden der Formel $R^2SCl$ nach folgendem Reaktionsschema herstellen :

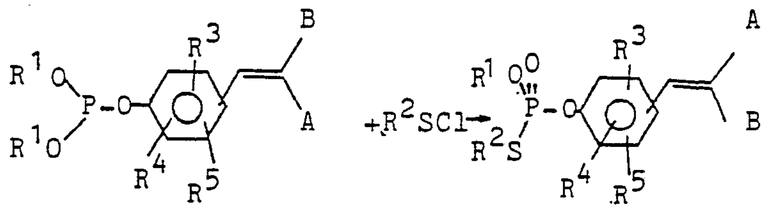

(IV) Ia

Ebenso sind die Dihalogenvinyl-phenyl-thiophosphorsäureester der Formel Ia durch Alkylierung von Phosphorsäureestersalzen der Formel V mit Alkylierungsmitteln der Formel $R^2Y$ zugänglich :

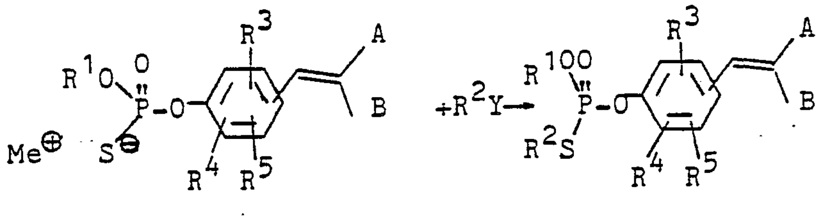

(V) Ia

Hierbei steht $Me^{\oplus}$ für ein vorzugsweise einwertiges Kation wie $Na^{\oplus}$, $K^{\oplus}$ oder $NH_4^{\oplus}$ und Y bedeutet Halogen oder einen Alkylsulfatrest.

Außerdem lassen sich Phosphorsäureesterdichloride der Formel IV mit Alkoholen und Mercaptanen der Formeln $R^1OH$ bzw. $R^2SH$ zu Verbindungen der Formel Ib umsetzen :

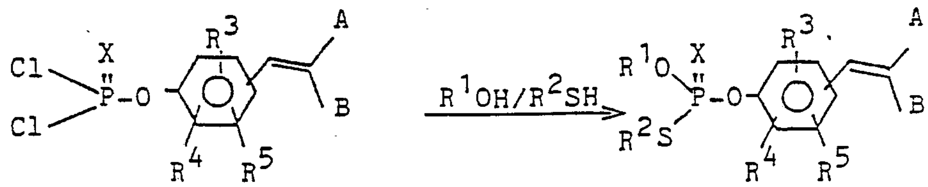


(VI) Ib

Dialkylphosphorsäureesterchloride sind bekannt und können nach bekannten Verfahren hergestellt werden (DE-A-2 642 982, J. Org. Chem. *30*, 3217 (1965), DE-A-2 552 945).

Dihalogenvinyl-phenole der Formel III können ebenfalls auf an sich bekannte Weise hergestellt werden.

Der nachstehende Reaktionsablauf wird z. B. durch Lewis-Säuren wie $AlCl_3$ bewirkt :

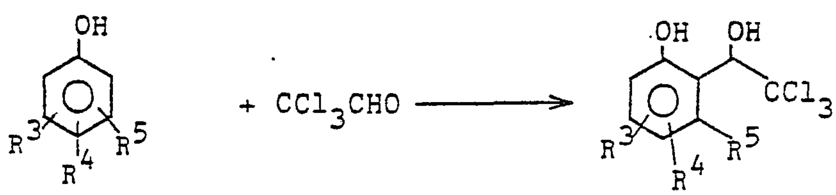

(VII) (VIII) (IX)

Eine geeignete Arbeitsvorschrift kann beispielsweise SYNTHESIS 1979, 824 entnommen werden.

Acetylierung von IX, Spaltung mit Zink in Eisessig (DE-A-2 633 551) und Verseifung mit NaOH führen über X, XI zu IIIa :

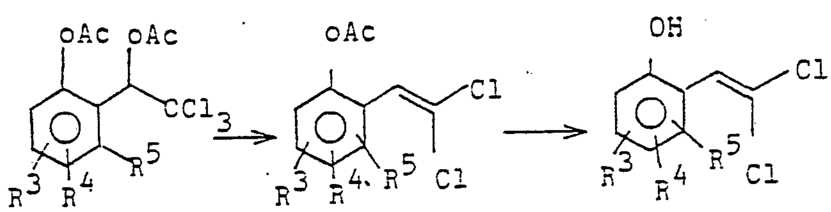

(X) (XI) IIIa

Die auf diese Weise nicht zugänglichen meta- und para-Dihalogenvinylphenole IIIb und IIIc können durch Umsetzung der mit 3,4-Dihydropyran geschützten meta- und para-Hydroxy-benzaldehyde XII beispielsweise mit Natriumhydrid und Chloroform zur Verbindung XIII mit anschließender Acetylierung und Abspaltung der Schutzgruppe mit Zink in Eisessig erhalten werden :

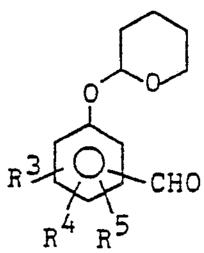
(XII)

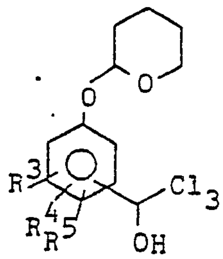
(XIII)

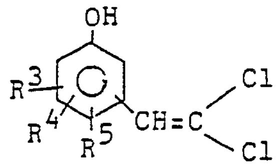
IIIb bzw. IIIc

Eine andere Möglichkeit zur Erlangung von Verbindungen der Formel III bietet die Umsetzung entsprechender Aldehyde XII mit Phosphoryliden der Formel XIV nach Wittig :

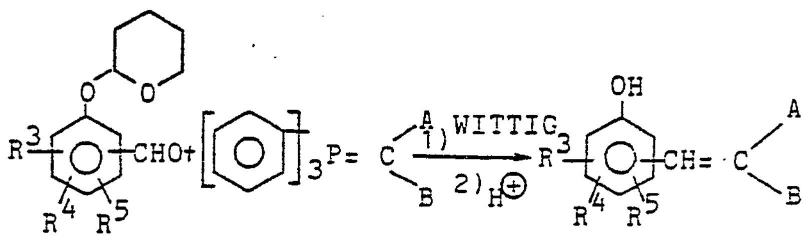


(XII) (XIV) (III)

Die Dihalogenvinylphenole III bzw. IIIa, IIIb bzw. IIIc können vor ihrer Weiterverarbeitung zu Phenolphosphorsäureester anderen Umsetzungen unterworfen werden, z. B. mittels Salpetersäure nitriert werden.

Herstellungsbeispiel für o-Dihalogenvinylphenole

a) 33,6 g (0,6 mol) Kaliumhydroxid und 67 g (0,6 mol) 4-Fluor-phenol werden in 300 ml Ethanol gelöst und zur Trockene eingeengt. Zu einer Suspension des erhaltenen Kaliumsalzes in 1 200 ml absol. Toluol werden 67 g (0,6 mol) 4-Fluor-phenol und portionsweise 79,2 g (0,6 mol) Aluminium-chlorid eingetragen. Nach zehnminütigem Rückfluß wird auf unter 20 °C abgekühlt und 177,6 g (1,2 mol) wasserfreies Chloral, gelöst in 600 ml absol. Toluol, allmählich zugesetzt. Man läßt über Nacht rühren und versetzt unter Eiskühlung mit 1,5 l gesättigter Ammoniumchloridlösung. Die Phasen werden getrennt, die wäßrige Phase dreimal mit Toluol extrahiert und die Extrakte vereinigt, gewaschen und eingeengt.

Man erhält als kristallinen Rückstand 4-Fluor-2-(2,2,2-Trichlor-1-hydroxyethyl)-phenol in einer Ausbeute von 91 g mit einem Fp = 116 bis 120 °C (nach Umkristallisation aus Toluol).

Analyse

| | | | | | |
|---|---|---|---|---|---|
| ber. | C 37,03 | H 2,33 | Cl 40,99 | F 7,32 | O 12,33 |
| gef. | C 37,4 | H 2,6 | Cl 41,2 | F 7,3 | O Rest |

b) 90 g (0,35 mol) 4-Fluor-2-(2,2,2-Trichlor-1-hydroxyethyl)-phenol werden in 800 ml absol. Ether gelöst und mit 70 g (0,7 mol) Triethylamin in 500 ml absol. Ether versetzt. Bei 0 bis 5 °C setzt man nach und nach 55 g (0,7 mol) Acetylchlorid in 300 ml absol. Ether zu. Nach dreistündigem Rühren bei RT wird abgesaugt und das Filtrat eingeengt.

Man erhält als kristallinen Rückstand 4-Fluor-2-(2,2,2-trichlor-1-acetoxy-ethyl)-acetoxybenzol mit einem Fp = 90 bis 95 °C in einer Ausbeute von 110 g.

Analyse

| | | | | | |
|---|---|---|---|---|---|
| ber. | C 41,42 | H 2,73 | Cl 30,96 | F 5,53 | O 18,63 |
| gef. | C 42,1 | H 3,0 | Cl 31,4 | F 5,5 | O Rest |

c) 107 g (0,31 mol) 4-Fluor-2-(2,2,2-trichlor-1-acetoxy-ethyl)-acetoxybenzol werden in 550 ml Eisessig suspendiert. Dazu trägt man portionsweise 23,5 g (0,36 mol) Zinkstaub ein. Man läßt 6 h bei 60 bis 70 °C rühren, kühlt ab, saugt vom Niederschlag ab und gießt das Filtrat in 1,5 l destilliertes Wasser. Man

extrahiert mehrmals mit Chloroform vereinigt die Extrakte, wäscht mit $NaHCO_3$-Lösung und Wasser, trocknet und engt ein.

Man erhält als kristallinen Rückstand 4-Fluor-2-(2,2-dichlorvinyl)-acetoxybenzol mit einem Fp = 47 bis 50 °C in einer Ausbeute von 73 g.

| Analyse | | | | | |
|---|---|---|---|---|---|
| ber. | C 48,22 | H 2,83 | Cl 28,47 | F 7,63 | O 12,85 |
| gef. | C 47,3 | H 2,9 | Cl 29,1 | F 7,5 | O Rest |

d) 68 g (0,275 mol) 4-Fluor-2-(2,2-dichlorvinyl)-acetoxybenzol in 100 ml absol. Ethanol werden zu 500 ml einer kalt gesättigten NaOH-Ethanollösung gegeben. Man läßt mehrere Stunden rühren und stellt danach unter Kühlen mit Essigsäure auf pH 4,7 ein. Man engt ein (starkes Schäumen), nimmt mit Wasser und Ether auf, extrahiert mehrmals mit Ether, vereinigt die Etherextrakte, wäscht mit $NaHCO_3$-Lösung und Wasser, trocknet über Natriumsulfat und engt ein. Man erhält als kristallinen Rückstand 4-Fluor-2-(2,2-dichlorvinyl)phenol mit einem Fp = 55 bis 57 °C in einer Ausbeute von 44 g.

| Analyse | | | | | |
|---|---|---|---|---|---|
| ber. | C 46,41 | H 2,43 | Cl 34,25 | F 9,18 | O 7,73 |
| gef. | C 46,8 | H 2,6 | Cl 34,1 | F 9,0 | O Rest |

Weiterverarbeitung eines Dichlorvinylphenols durch Einführung eines Substituenten

e) 9,5 g (0,05 mol) 2-(2',2'-Dichlorvinyl)phenol werden in 60 ml Acetanhydrid gelöst. Bei 5 bis 10 °C werden innerhalb von 1 h eine Mischung von 6,2 g 65 % Salpetersäure und 6,4 g Schwefelsäure konzentriert eingetropft. Nach zweistündigem Rühren bei 10 bis 15 °C wird in 0,5 l Wasser gegossen, ausgeethert, die Etherlösung vereinigt, mit Wasser gewaschen, getrocknet und eingeengt.

Man erhält nach längerem Stehenlassen als kristallinen Rückstand 4-Nitro-2-(2,2-dichlorvinyl)-acetoxybenzol mit einem Fp = 80 bis 90 °C in einer Ausbeute von 10 g.

| Analyse | | | | | |
|---|---|---|---|---|---|
| ber. | C 43,51 | H 2,56 | N 5,07 | Cl 25,68 | O 23,18 |
| gef. | C 43,8 | H 2,6 | N 4,9 | Cl 25,8 | O Rest |

f) 11 g (0,04 mol) 4-Nitro-2-(2,2-dichlorvinyl)acetoxybenzol werden mit 50 ml 4 % HCl-Lösung unter Stickstoffspülung 4 h unter Rückfluß erhitzt, abgekühlt und dreimal ausgeethert. Die Etherextrakte werden vereinigt, mit $NaHCO_3$-Lösung und Wasser gewaschen, über $Na_2SO_4$ wfr. getrocknet und eingeengt.

Man erhält nach Filtration über Kieselgel mit Toluol als kristallinen Rückstand 4-Nitro-2-(2,2-dichlorvinyl)phenol in 4,0 g Ausbeute mit einem Fp = 107 bis 115 °C.

| Analyse | | | | | |
|---|---|---|---|---|---|
| ber. | C 41,06 | H 2,15 | N 5,98 | Cl 30,30 | O 20,51 |
| gef. | C 41,2 | H 2,1 | N 5,9 | Cl 29,9 | O 20,7 |

Herstellung für m- und p-Dichlorvinylphenole

a) 24,4 g (0,2 mol) 3-Hydroxy-benzaldehyd werden in 400 ml absol. Tetrahydrofuran gelöst und mit einer Spatelspitze p-Toluolsulfonsäure versetzt. Danach werden 34 g (0,4 mol) 3,4-Dihydropyran bei RT eingetropft. Nach vierstündigem Rühren wird mit zehnprozentiger Kaliumhydroxid-Lösung gewaschen, über Kaliumcarbonat getrocknet und im Rotationsverdampfer eingeengt.

Man erhält 3-(Tetrahydropyranyloxy)benzaldehyd in einer Ausbeute von 40,0 g in Form eines Öls (nach einer Filtration der toluolischen Lösung über Kieselgel) mit einem Brechungsindex $n_D^{20}$ = 1,546 0.

| Analyse | | | |
|---|---|---|---|
| ber. | C 69,89 | H 6,84 | O 23,27 |
| gef. | C 69,2 | H 6,8 | O Rest |

b) Eine Mischung von 27 g (0,13 mol) 3-(Tetrahydropyranyloxy)-benzaldehyd, 17,3 g (0,144 Mol) wasserfreiem Chloroform und 30 ml Tetrahydrofuran wird unter Stickstoff bei RT zu einer Suspension von 4,6 g (0,144 Mol) Natriumhydrid in 130 ml Tetrahydrofuran nach und nach zugesetzt und 1 h bei 40 °C nachgerührt. Nach Abkühlen auf unter 5 °C werden 10,4 g (0,13 Mol) Acetylchlorid in 70 ml Tetrahydrofuran zugesetzt. Man läßt über Nacht rühren, versetzt mit Wasser und extrahiert mit Ether. Die Extrakte werden mit einer $NaHCO_3$-Lösung und Wasser gewaschen, getrocknet, eingeengt und in Toluol aufgenommen.

Nach Filtration der toluolischen Lösung über Kieselgel und Entfernen des Toluols erhält man 20 g kristallines 3-(Tetrahydropyranyloxy)-1-(2,2,2-trichlor-1-acetoxyethyl)-benzol mit einem Fp = 90 bis 95 °C.

Analyse

| | | | | |
|---|---|---|---|---|
| ber. | C 49,00 | H 4,66 | Cl 28,93 | O 17,41 |
| gef. | C 49,6 | H 4,9 | Cl 28,8 | O 17,1 |

c) 22 g (0,06 mol) 3-(Tetrahydropyranyloxy)-1-(2,2,2-trichlor-1-acetoxyethyl)-benzol werden in 80 ml Eisessig gelöst. Dazu werden 4,6 g (0,07 mol) Zinkstaub portionsweise gegeben. Man erhitzt 3 h auf 60 bis 70 °C. Nach dem Abkühlen und Filtrieren wird in 1,5 l Wasser eingetragen und mit Ether extrahiet. Die Extrakte werden vereinigt, dreimal mit $NaHCO_3$-Lösung und Wasser gewaschen, getrocknet, eingeengt und mit Toluol aufgenommen. Nach Filtration über Kieselgel erhält man 8,0 g 4-Dichlorvinyl-phenol als wachsartiges Produkt.

Analyse

| | | | | |
|---|---|---|---|---|
| ber. | C 50,83 | H 3,20 | Cl 37,51 | O 8,46 |
| gef. | C 51,2 | H 3,5 | Cl 36,5 | O 8,8 |

Die in den nachstehenden Tabellen 1, 2 und 3 beschriebenen Beispiele von (2,2-Dihalogenvinyl)phenolen wurden unter entsprechender Abwandlung der vorstehenden praktischen Angaben nach einem der anderen, oben beschriebenen Verfahren hergestellt oder können — soweit keine physikalischen Angaben gemacht werden — aus entsprechend gebauten Rohstoffen erhalten werden.

Tabelle 1

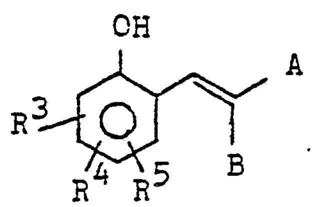

| A | B | $R^3$ | $R^4$ | $R^5$ | $n_D$/Fp |
|---|---|---|---|---|---|
| Cl | Cl | 4-Br | H | H | Fp = 64 - 67°C |
| Cl | Cl | H | H | H | Fp = 47 - 50°C |
| Cl | Cl | 4-$OCH_3$ | H | H | $n_D^{23}$= 1,5900 |
| Br | Br | 4-F | H | H | $n_D^{22}$= 1,6195 |
| Cl | Cl | 4-$SCH_3$ | H | H | $n_D^{20}$= 1,6458 |
| Cl | Cl | 4-Cl | H | H | Fp = 61 - 65°C |
| Cl | Cl | 4-$CH_3$ | H | H | Fp = 42 - 44°C |
| Br | Br | H | H | H | |
| F | F | H | H | H | |
| F | F | 4-F | H | H | |
| F | F | 4-F | H | H | |
| Br | Br | 4-Br | H | H | |
| $CF_3$ | $CF_3$ | H | H | H | |
| $CCl_3$ | $CCl_3$ | H | H | H | |
| F | $CCl_3$ | H | H | H | |

(Fortsetzung)

| A | B | $R^3$ | $R^4$ | $R^5$ | $n_D$/Fp |
|---|---|---|---|---|---|
| Cl | $CF_3$ | H | H | H | |
| F | $CF_3$ | H | H | H | |
| Cl | $CCl_3$ | H | H | H | |
| Br | F | H | H | H | |
| F | Cl | H | H | H | |
| Br | Cl | H | H | H | |
| Cl | Cl | 4-CN | H | H | |

Tabelle 2

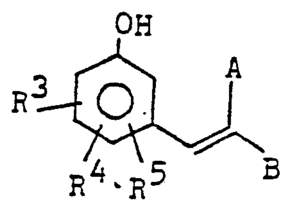

| A | B | $R^3$ | $R^4$ | $R^5$ | $n_D$/Fp |
|---|---|---|---|---|---|
| F | F | H | H | H | |
| Br | Br | H | H | H | |
| Cl | Cl | 4-F | H | H | |
| Cl | Cl | 4-Cl | H | H | |
| Cl | Cl | 4-Br | H | H | |
| Cl | Cl | $6-OCH_3$ | H | H | |
| F | F | $6-OCH_3$ | H | H | |
| Br | Br | $6-OCH_3$ | H | H | |
| $CF_3$ | $CF_3$ | H | H | H | |
| $CCl_3$ | $CCl_3$ | H | H | H | |
| F | $CF_3$ | H | H | H | |
| F | $CCl_3$ | H | H | H | |
| Cl | $CF_3$ | H | H | H | |
| Cl | $CCl_3$ | H | H | H | |
| Br | F | H | H | H | |
| Br | Cl | H | H | H | |
| F | Cl | H | H | H | |

Tabelle 3

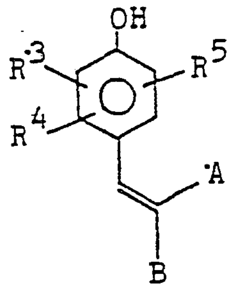

| A | B | $R^3$ | $R^4$ | $R^5$ | $n_D$/Fp |
|---|---|---|---|---|---|
| Cl | Cl | H | H | H | Fp. 88 - 91°C |
| Cl | Cl | 2-$OC_2H_5$ | H | H | |
| Cl | Cl | 3-$OCH_3$ | H | H | |
| Cl | Cl | 2-$OCH_3$ | 6-Br | H | |
| Cl | Cl | 2-Br | 6-Br | H | |
| Cl | Cl | 2-J | 6-J | H | |
| F | F | H | H | H | |
| Br | Br | H | H | H | |
| $CF_3$ | $CF_3$ | H | H | H | |
| $CCl_3$ | $CCl_3$ | H | H | H | |
| $CCl_3$ | F | H | H | H | |
| $CCl_3$ | Cl | H | H | H | |
| $CF_3$ | F | H | H | H | |
| $CF_3$ | Cl | H | H | H | |
| Br | F | H | H | H | |
| F | Cl | H | H | H | |
| Cl | Br | H | H | H | |

Herstellbeispiel für einen Phosphorester-Wirkstoff

8,0 g (0,03 mol) 4-Br-2(2,2-dichlorvinyl)-phenol werden in 100 ml Methanol gelöst, mit 5,4 g (0,03 mol) 30 % Natriummethylatlösung in Methanol versetzt und eingeengt. Das Natriumsalz wird mit 100 ml absol. Aceton aufgenommen. Zu dieser Lösung tropft man 5,0 g (0,031 mol) O,O-Dimethylthiophosphorsäurechlorid in 10 ml absol. Aceton. Es wird über Nacht gerührt, mit Wasser versetzt und mit Ether extrahiert. Die Extrakte werden vereinigt, mit Wasser gewaschen, getrocknet, eingeengt. Nach Chromatographie an einer Kieselgelsäule mit Toluol als Elutionsmittel erhält man 7,2 g O,O-Dimethyl-O- 4-Br-2-(2-dichlorvinyl)-phenyl-thiophosphat (Wirkstoffbeispiel Nr. 1) als Öl mit einem Brechungsindex $n_D^{23}$ = 1,590 0.

Analyse

| | | | | | | | |
|---|---|---|---|---|---|---|---|
| ber. | C 30,64 | H 2,57 | O 12,24 | S 8,18 | Cl 18,09 | Br 20,38 | P 7,90 |
| gef. | C 30,9 | H 2,6 | O 12,0 | S 8,6 | Cl 18,4 | Br 20,5 | P 7,7 |

Die in den nachstehenden Tabellen 4, 5 und 6 aufgeführten Phosphorsäureester wurden hergestellt, soweit physikalische Merkmale (Schmelzpunkt/Brechungsindex) angegeben sind ; sie weisen i. a. eine gute oder überdurchschnittlich gute insektizide Wirkung auf. Die nicht weiter erläuterten Verbindungen sind Beispiele für erfindungsgemäße Verbindungen ; aufgrund ihrer strukturellen Ähnlichkeit mit den als wirksam gefundenen Verbindungen lassen sie eine ähnliche Eignung als Wirkstoffe erwarten.

Tabelle 4

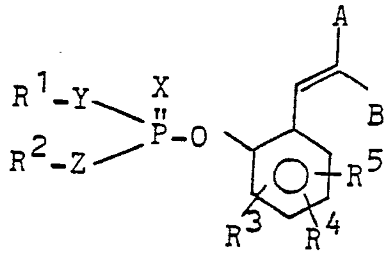

| Nr. | A | B | X | Y | Z | $R^1$ | $R^2$ | $R^3$ | $R^4$ | $R^5$ | $n_D$/Fp |
|---|---|---|---|---|---|---|---|---|---|---|---|
| 2 | Cl | Cl | S | O | O | $C_2H_5$ | $C_2H_5$ | 4-F | H | H | $n_D^{23}$= 1,5324 |
| 3 | Cl | Cl | S | O | O | $CH_3$ | $CH_3$ | 4-F | H | H | $n_D^{23}$= 1,5545 |
| 4 | Cl | Cl | S | O | S | $C_2H_5$ | $C_2H_5$ | 4-F | H | H | $n_D^{23}$= 1,5713 |
| 5 | Cl | Cl | S | O | S | $C_2H_5$ | $C_3H_7$ | 4-F | H | H | $n_D^{23}$= 1,5658 |
| 6 | Cl | Cl | O | O | S | $C_2H_5$ | $C_3H_7$ | 4-F | H | H | $n_D^{23}$= 1,5399 |
| 7 | Cl | Cl | S | N | N | $(CH_3)_2$ | $(CH_3)_2$ | 4-F | H | H | Fp = 73-77°C |
| 8 | Cl | Cl | O | O | S | $C_2H_5$ | $C_3H_7$ | 4-Br | H | H | $n_D^{23}$= 1,5722 |
| 9 | Cl | Cl | S | O | O | $C_2H_5$ | $C_2H_5$ | 4-Br | H | H | $n_D^{23}$= 1,5683 |
| 10 | Cl | Cl | S | O | S | $C_2H_5$ | $C_2H_5$ | 4-Br | H | H | $n_D^{23}$= 1,6018 |
| 11 | Cl | Cl | S | N | N | $(CH_3)_2$ | $(CH_3)_2$ | 4-Br | H | H | Fp = 124-130°C |
| 12 | Cl | Cl | S | O | O | $C_2H_5$ | $C_2H_5$ | 4-$OCH_3$ | H | H | $n_D^{22}$= 1,5504 |
| 13 | Cl | Cl | O | O | S | $C_2H_5$ | $C_3H_7$ | 4-$OCH_3$ | H | H | $n_D^{22}$= 1,5603 |
| 14 | Cl | Cl | S | O | S | $C_2H_5$ | $C_2H_5$ | 4-$OCH_3$ | H | H | $n_D^{22}$= 1,5795 |
| 15 | Cl | Cl | O | O | S | $C_2H_5$ | $C_3H_7$ | 4-$CH_3$ | H | H | $n_D^{23}$= 1,5503 |
| 16 | Cl | Cl | S | O | O | $C_2H_5$ | $C_2H_5$ | 4-$CH_3$ | H | H | $n_D^{23}$= 1,5465 |
| 17 | Cl | Cl | S | O | O | $CH_3$ | $CH_3$ | 4-$CH_3$ | H | H | $n_D^{23}$= 1,5629 |
| 18 | Cl | Cl | O | O | S | $C_2H_5$ | $C_3H_7$ | 4-Cl | H | H | $n_D^{23}$= 1,5583 |
| 19 | Cl | Cl | S | O | O | $C_2H_5$ | $C_2H_5$ | 4-Cl | H | H | $n_D^{23}$= 1,5555 |
| 20 | Cl | Cl | S | O | N | $C_2H_5$ | i-$C_3H_7$ | 4-Cl | H | H | $n_D^{20}$= 1,5670 |
| 21 | Cl | Cl | S | O | O | $CH_3$ | $CH_3$ | 4-Cl | H | H | viskoses Öl |
| 22 | Cl | Cl | O | O | S | $C_2H_5$ | $C_3H_7$ | H | H | H | $n_D^{23}$= 1,5575 |
| 23 | Cl | Cl | S | O | N | $C_2H_5$ | i-$C_3H_7$ | H | H | H | $n_D^{23}$= 1,5440 |
| 24 | Cl | Cl | S | O | O | $C_2H_5$ | $C_2H_5$ | H | H | H | $n_D^{26}$= 1,5466 |
| 25 | Cl | Cl | S | O | O | $CH_3$ | $CH_3$ | H | H | H | $n_D^{26}$= 1,5674 |
| 26 | Cl | Cl | O | O | S | $C_2H_5$ | $C_3H_7$ | 4-$SCH_3$ | H | H | $n_D^{20}$= 1,5812 |
| 27 | Cl | Cl | O | O | S | $C_2H_5$ | $CH_3$-O-$C_2H_4$ | 4-$SCH_3$ | H | H | $n_D^{20}$= 1,5771 |

Tabelle 4 (Fortsetzung)

| Nr. | A | B | X | Y | Z | $R^1$ | $R^2$ | $R^3$ | $R^4$ | $R^5$ | $n_D$/Fp |
|---|---|---|---|---|---|---|---|---|---|---|---|
| 28 | Cl | Cl | O | O | S | $C_2H_5$ | $i-C_3H_7-O-C_2H_4$ | $4-SCH_3$ | H | H | $n_D^{23}= 1,5650$ |
| 29 | Cl | Cl | O | O | S | $C_2H_5$ | $sec-C_4H_9$ | $4-SCH_3$ | H | H | $n_D^{20}= 1,5695$ |
| 30 | Br | Br | O | O | S | $C_2H_5$ | $i-C_3H_7-O-C_2H_4$ | H | H | H | $n_D^{18}= 1,5637$ |
| 31 | Br | Br | O | O | S | $C_2H_5$ | $C_3H_7$ | H | H | H | |
| 32 | Br | Br | O | O | S | $C_2H_5$ | $C_3H_7$ | 4-Br | H | H | |
| 33 | Br | Br | O | O | S | $C_2H_5$ | $C_3H_7$ | 4-F | H | H | |
| 34 | F | F | O | O | S | $C_2H_5$ | $C_3H_7$ | H | H | H | |
| 35 | F | F | O | O | S | $C_2H_5$ | $C_3H_7$ | 4-F | H | H | |
| 36 | F | F | O | O | S | $C_2H_5$ | $C_3H_7$ | 4-Br | H | H | |
| 37 | Br | Cl | O | O | S | $C_2H_5$ | $C_3H_7$ | 4-Br | H | H | |
| 38 | F | Cl | O | O | S | $C_2H_5$ | $C_3H_7$ | 4-Br | H | H | |
| 39 | Br | F | O | O | S | $C_2H_5$ | $C_3H_7$ | 4-Br | H | H | |
| 40 | $Cl_3$ | $Cl_3$ | O | O | S | $C_2H_5$ | $C_3H_7$ | 4-F | H | H | |
| 41 | $CF_3$ | $CF_3$ | O | O | S | $C_2H_5$ | $C_3H_7$ | 4-F | H | H | |
| 42 | Cl | $CCl_3$ | O | O | S | $C_2H_5$ | $C_3H_7$ | 4-F | H | H | |
| 43 | F | $CCl_3$ | O | O | S | $C_2H_5$ | $C_3H_7$ | 4-F | H | H | |
| 44 | Cl | $CF_3$ | O | O | S | $C_2H_5$ | $C_3H_7$ | 4-F | H | H | |
| 45 | F | $CF_3$ | O | O | S | $C_2H_5$ | $C_3H_7$ | 4-F | H | H | |
| 46 | Cl | Cl | O | O | S | $C_2H_5$ | $sec-C_4H_9$ | $4-CF_3$ | H | H | |
| 47 | Cl | Cl | O | O | S | $C_2H_5$ | $sec-C_4H_9$ | $4-CCl_3$ | H | H | |
| 48 | Cl | Cl | O | O | S | $C_2H_5$ | $sec-C_4H_9$ | $4-OCF_3$ | H | H | |
| 49 | Cl | Cl | S | O | S | $C_2H_5$ | $C_3H_7$ | $4-NO_2$ | H | H | $n_D^{24}= 1.5910$ |
| 50 | Cl | Cl | O | O | S | $C_2H_5$ | $C_3H_7$ | 4-CN | H | H | |

Tabelle 5

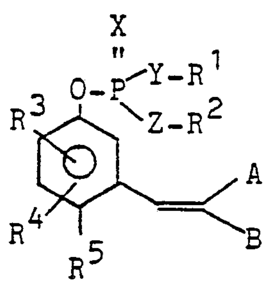

| Nr. | A | B | X | Y | Z | $R^1$ | $R^2$ | $R^3$ | $R^4$ | $R^5$ | $n_D$/Fp |
|---|---|---|---|---|---|---|---|---|---|---|---|
| 51 | Cl | Cl | O | O | S | $C_2H_5$ | $CH_3-O-C_2H_4$ | H | H | H | $n_D^{21}= 1,5568$ |
| 52 | Cl | Cl | O | O | S | $C_2H_5$ | $sec-C_4H_9$ | H | H | H | $n_D^{20}= 1,5520$ |
| 53 | Cl | Cl | O | O | S | $C_2H_5$ | $C_3H_7$ | $6-CF_3$ | H | H | |
| 54 | Cl | Cl | O | O | S | $C_2H_5$ | $C_3H_7$ | $4-OCF_3$ | H | H | |

(Fortsetzung)

| Nr. | A | B | X | Y | Z | $R^1$ | $R^2$ | $R^3$ | $R^4$ | $R^5$ | $n_D$/Fp |
|---|---|---|---|---|---|---|---|---|---|---|---|
| 55 | Br | Br | O | O | S | $C_2H_5$ | $C_3H_7$ | H | H | H | |
| 56 | Br | Br | O | O | S | $C_2H_5$ | $C_3H_7$ | 4-Br | H | H | |
| 57 | Br | Br | O | O | S | $C_2H_5$ | $C_3H_7$ | 2-F | H | H | |
| 58 | F | F | O | O | S | $C_2H_5$ | $C_3H_7$ | H | H | H | |
| 59 | F | F | O | O | S | $C_2H_5$ | $C_3H_7$ | 2-F | H | H | |
| 60 | F | F | O | O | S | $C_2H_5$ | $C_3H_7$ | 2-Br | H | H | |
| 61 | Br | Cl | O | O | S | $C_2H_5$ | $C_3H_7$ | 4-Br | H | H | |
| 62 | F | Cl | O | O | S | $C_2H_5$ | $C_3H_7$ | 4-Br | H | H | |
| 63 | Br | F | O | O | S | $C_2H_5$ | $C_3H_7$ | 4-Br | H | H | |
| 64 | $CCl_3$ | $CCl_3$ | O | O | S | $C_2H_5$ | $C_3H_7$ | 4-F | H | H | |
| 65 | $CF_3$ | $CF_3$ | O | O | S | $C_2H_5$ | $C_3H_7$ | 2-F | H | H | |
| 66 | Cl | $CCl_3$ | O | O | S | $C_2H_5$ | $C_3H_7$ | 2-F | H | H | |
| 67 | F | $CCl_3$ | O | O | S | $C_2H_5$ | $C_3H_7$ | 4-F | H | H | |
| 68 | Cl | $CF_3$ | O | O | S | $C_2H_5$ | $C_3H_7$ | 2-F | H | H | |
| 69 | F | $CF_3$ | O | O | S | $C_2H_5$ | $C_3H_7$ | 4-F | H | H | |
| 70 | Cl | Cl | O | O | S | $C_2H_5$ | sec-$C_4H_9$ | 6-$OCH_3$ | H | H | |
| 71 | Cl | Cl | O | O | S | $C_2H_5$ | i-$C_4H_9$ | H | H | H | |

Tabelle 6

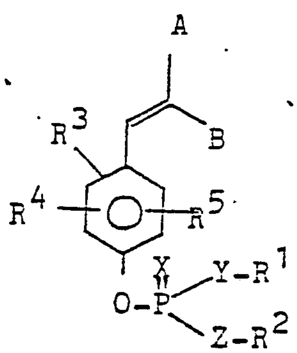

| Nr. | A | B | X | Y | Z | $R^1$ | $R^2$ | $R^3$ | $R^4$ | $R^5$ | $n_D$/Fp |
|---|---|---|---|---|---|---|---|---|---|---|---|
| 72 | Cl | Cl | O | O | S | $C_2H_5$ | $C_3H_7$ | H | H | H | $n_D^{23}$= 1,5616 |
| 73 | Cl | Cl | O | O | S | $C_2H_5$ | sec-$C_4H_9$ | H | H | H | $n_D^{23}$= 1,5590 |
| 74 | Cl | Cl | O | O | S | $C_2H_5$ | i-$C_4H_9$ | H | H | H | $n_D^{23}$= 1,5558 |
| 75 | Cl | Cl | O | O | S | $C_2H_5$ | $C_3H_7$ | 2-$CF_3$ | H | H | |
| 76 | Cl | Cl | O | O | S | $C_2H_5$ | sec-$C_4H_9$ | 2-$OCF_3$ | H | H | |
| 77 | Br | Br | O | O | S | $C_2H_5$ | $C_3H_7$ | H | H | H | |
| 78 | Br | Br | O | O | S | $C_2H_5$ | $C_3H_7$ | 2-Br | H | H | |
| 79 | Br | Br | O | O | S | $C_2H_5$ | $C_3H_7$ | 2-F | H | H | |

(Fortsetzung)

| Nr. | A | B | X | Y | Z | $R^1$ | $R^2$ | $R^3$ | $R^4$ | $R^5$ | $n_D$/Fp |
|---|---|---|---|---|---|---|---|---|---|---|---|
| 80 | F | F | O | O | S | $C_2H_5$ | $C_3H_7$ | H | H | H | |
| 81 | F | F | O | O | S | $C_2H_5$ | $C_3H_7$ | 2-F | H | H | |
| 82 | F | F | O | O | S | $C_2H_5$ | $C_3H_7$ | 2-Br | H | H | |
| 83 | Br | Cl | O | O | S | $C_2H_5$ | $C_3H_7$ | 2-Br | H | H | |
| 84 | F | Cl | O | O | S | $C_2H_5$ | $C_3H_7$ | 2-Br | H | H | |
| 85 | Br | F | O | O | S | $C_2H_5$ | $C_3H_7$ | 2-Br | H | H | |
| 86 | $CCl_3$ | $CCl_3$ | O | O | S | $C_2H_5$ | $C_3H_7$ | 2-F | H | H | |
| 87 | $CF_3$ | $CF_3$ | O | O | S | $C_2H_5$ | $C_3H_7$ | 2-F | H | H | |
| 88 | Cl | $CCl_3$ | O | O | S | $C_2H_5$ | $C_3H_7$ | 2-F | H | H | |
| 89 | F | $CCl_3$ | O | O | S | $C_2H_5$ | $C_3H_7$ | 2-F | H | H | |
| 90 | Cl | $CF_3$ | O | O | S | $C_2H_5$ | $C_3H_7$ | 2-F | H | H | |
| 91 | F | $CF_3$ | O | O | S | $C_2H_5$ | $C_3H_7$ | 2-F | H | H | |
| 92 | Cl | Cl | O | O | S | $C_2H_5$ | sec-$C_4H_9$ | 3-$OCH_3$ | H | H | |
| 93 | Cl | Cl | O | O | S | $C_2H_5$ | sec-$C_4H_9$ | 2-Br | 6-Br | H | |
| 94 | Cl | Cl | O | O | S | $C_2H_5$ | sec-$C_4H_9$ | 2-J | 6-J | H | |
| 95 | Cl | Cl | O | O | S | $C_2H_5$ | $CH_3$-O-$C_2H_4$ | H | H | H | |

Die vorstehend aufgeführten und andere erfindungsgemäße Wirkstoffe werden auf die für Phosphorsäureester übliche Weise angewendet. Hinweise zur Formulierung, Anwendungstechnik und Wirkungsweise und Angaben über geeignete Mischungspartner zur Erzielung synergistischer und anderer vorteilhafter Wirkungen können beispielsweise der DE-A-3 039 080 oder der entsprechenden EP-A-0 050 219 entnommen werden.

Ein besonders wichtiges Anwendungsgebiet der — an sich breit wirksamen — Insektizide ist der Einsatz gegen Raupen von Schadschmetterlingen, die in der vorgenannten Publikation aufgeführt sind.

In den nachstehenden Anwendungsbeispielen wurde als Vergleichsmittel das Handelsprodukt Chlorphenvinphos verwendet.

Anwendungsbeispiel 1

Kontaktwirkung auf Schaben (Blatta orientalis)

Der Boden eines 1-1-Weckglases wird mit der acetonischen Lösung des Wirkstoffes behandelt.

Nach Verdunsten des Lösungsmittels besetzt man jedes Glas mit 5 adulten Schaben.

Die Mortalitätsrate wird nach 48 Stunden bestimmt.

Bei diesem Test erwiesen sich die Wirkstoffe der Beispiele 5, 6, 8, 15, 18, 22, 29 und 51 in einer Menge von 0,1 mg oder weniger als voll wirksam, d. h. sie erzielten 100 %ige Mortalität, während das Vergleichsmittel eine nur ungenügende Wirksamkeit besaß. 100 %ige Mortalität konnte mit dem Vergleichsmittel erst mit 0,25 mg erreicht werden.

Anwendungsbeispiel 2

200 ml Leitungswasser werden mit der Wirkstoffaufbereitung versetzt und darauf mit 30 bis 40 Moskito-Larven im 4. Larvenstadium besetzt.

Die Versuchstemperatur beträgt 20 °C. Nach 24 Stunden wird die Wirkung ermittelt.

Bei diesem Test erzielten die Wirkstoffe der Beispiele 5, 6, 8, 13, 15, 18 und 22 eine wenigstens 5-fach bessere Wirkung als das Vergleichsmittel.

Anwendungsbeispiel 3

Petrischalen von 10 cm Durchmesser werden mit 1 ml der acetonischen Wirkstofflösung ausgekleidet.

Nach Verdunsten des Lösungsmittels besetzt man die Schalen mit je 20 Larven des vorletzten Stadiums und registriert die Wirkung nach 24 Stunden.

Bei diesem Test erzielten die Wirkstoffe der Beispiele 6, 8, 18, 22, 27 und 51 eine 2- bis 10-fach bessere Wirkung als das Vergleichsmittel.

Anwendungsbeispiel 4

Plutella maculipennis, Kohlschaben-Raupen ; Fraß- und Kontaktwirkung

Blätter von jungen Kohlpflanzen werden 3 Sekunden in der wäßrige Wirkstoffemulsion getaucht und nach kurzem Abtropfen auf einen angefeuchteten Filter in eine Petrischale gelegt. Das Blatt wird darauf mit 10 Raupen des 4. Stadiums belegt.

Nach 48 Stunden beurteilt man die Wirkung.

Bei diesem Test erzielten die Wirkstoffe der Beispiele 5, 6, 8, 13, 18, 22, 26 und 51 eine 2- bis 10-fach bessere Wirkung als das Vergleichsmittel.

Anwendungsbeispiel 5

Kontaktwirkung auf Blattläuse (Aphis fabae) Spritzversuch

Getopfte Bohnenpflanzen (Vicia faba) mit starken Blattlauskolonien werden in der Spritzkammer mit den wäßrigen Wirkstoffaufbereitungen tropfnaß gespritzt.

Die Auswertung erfolgt nach 24 Stunden.

Bei diesem Test erzielten die Wirkstoffe 6, 13, 15 und 18 eine mindestens 2-fach bessere Wirkung als das Vergleichsmittel.

Anwendungsbeispiel 6

Wirkung auf Spinnmilben (Tetranychus telarius) (Test A)

Getopfte Buschbohnen, die das erste Folgeblattpaar entwickelt haben und einen starken Besatz aller Stadien der Spinnmilbe Tetranychus telarius tragen, werden in der Spritzkabine mit der wäßrigen Wirkstoffaufbereitung tropfnaß gespritzt. Die Pflanzen kommen dazu auf einen Drehteller und werden von allen Seiten mit 50 ml Spritzbrühe besprüht. Der Sprühvorgang dauert ca. 22 Sekunden.

Nach 8 Tagen werden die Pflanzen auf lebende Spinnmilben untersucht.

Bei diesem Test erzielten die Wirkstoffe der Beispiele 5, 6, 8, 18 und 22 eine 4- bis 20-fach bessere Wirkung als das Vergleichsmittel.

Anwendungsbeispiel 7

Wirkung auf Wurzelgallennematoden (Meloidogyne incognita)

Je 300 g Komposterde werden mit 30 ml der wäßrigen Wirkstoffaufbereitung innig vermischt und in die Plastiktöpfe gefüllt. Darauf bepflanzt man die Töpfe mit Tomatensetzlingen und hält diese unter Gewächshausbedingungen bei 22 bis 24 °C.

Nach 6 bis 8 Wochen untersucht man die Wurzeln auf Gallenbildung.

Bei diesem Test erzielten die Wirkstoffe der Beispiele 6, 8, 18 und 22 eine Wirkung bei einer Konzentration von 0,1 % oder weniger, während sich das Vergleichsmittel als unwirksam erwies.

**Patentansprüche**

1. Dihalogenvinylphenyl-phosphorsäureester der Formel I,

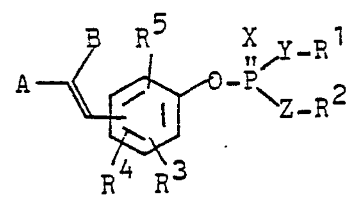

(I)

in der

A, B unabhängig voneinander Chlor, Fluor, Brom oder Trihalogenmethyl.

X Sauerstoff oder Schwefel

Y, Z unabhängig voneinander Sauerstoff, Schwefel oder —NH—

$R^1$ Alkyl mit 1 bis 3 Kohlenstoffatomen,

$R^2$ Alkyl, Halogenalkyl, Alkoxyalkyl, Alkylthioalkyl mit 1 bis 6 Kohlenstoffatomen

$R^3$, $R^4$, $R^5$ unabhängig voneinander Wasserstoff, Halogen, Nitro, Alkyl, Cyano, Alkoxy, Alkoxyalkoxy, Alkylthioalkylthio, Alkoxyalkyl, Alkylthioalkyl, Alkylthio, Trihalogenmethyl oder Trihalogenmethoxy. bedeuten.

2. Verfahren zur Herstellung von Verbindungen gemäß Anspruch 1, dadurch gekennzeichnet, daß man eine entsprechende Phosphor-halogen-Verbindung (II)

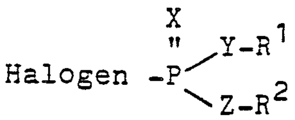

(II)

in Gegenwart eines säurebindenden Mittels mit einem entsprechenden Phenol (III)

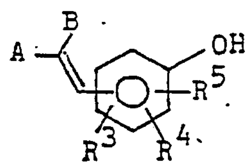

(III)

umsetzt.

3. Mittel zur Bekämpfung von Schädlingen, enthaltend einen Phosphorsäureester gemäß Anspruch 1 und wenigstens einen festen oder flüssigen Trägerstoff und gegebenenfalls eine synergistisch wirkende Verbindung.

4. Verwendung der Verbindungen gemäß Anspruch 1 oder der Mittel gemäß Anspruch 3 zur Bekämpfung von tierischen Schädlingen, indem man Verbindung bzw. Mittel auf die Schädlinge bzw. deren Lebensraum einwirken läßt.

**Claims**

1. A dihalovinylphenyl phosphate of the formula I

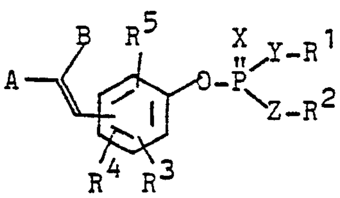

(I)

where

A and B independently of one another are chlorine, fluorine, bromine or trihalomethyl,

X is oxygen or sulfur,

Y and Z independently of one another are oxygen, sulfur or —NH—,

$R^1$ is alkyl of 1 to 3 carbon atoms,

$R^2$ is alkyl, haloalkyl, alkoxyalkyl, alkylthioalkyl of 1 to 6 carbon atoms, and

$R^3$, $R^4$ and $R^5$ independently of one another are hydrogen, halogen, nitro, alkyl, cyano, alkoxy, alkoxyalkoxy, alkylthioalkylthio, alkoxyalkyl, alkylthioalkyl, alkylthio, trihalomethyl or trihalomethoxy.

2. A process for the preparation of a compound as claimed in claim 1, wherein an appropriate phosphorus-halogen compound (II)

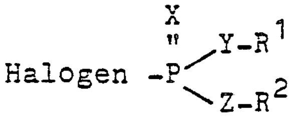

(II)

is reacted in the presence of an acid binder with an appropriate phenol (III)

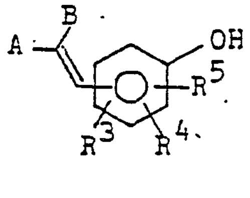

(III)

3. An agent for combating pests, containing a phosphoric acid ester as claimed in claim 1, at least one solid or liquid carrier and, if desired, a compound having a synergistic action.

4. The use of a compound as claimed in claim 1 or an agent as claimed in claim 3 for combating animal pests by allowing the compound or agent to act on the pests or their habitat.

**Revendications**

1. Ester d'acide dihalogénovinylphénylphosphorique de formule I

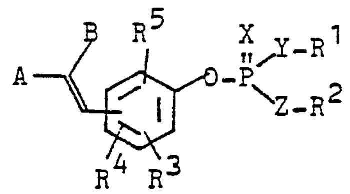

(I)

dans laquelle

A, B représentent, indépendamment l'un de l'autre, chlore fluor, brome ou trihalogénométhyle,

X oxygène ou soufre

Y, Z, indépendamment l'un de l'autre, oxygène, soufre ou —NH—

$R^1$ alkyle ayant 1 à 3 atomes de carbone

$R^2$ alkyle, halogènalkyle, alcoxyalkyle, alkylthioalkyle ayant 1 à 6 atomes de carbone

$R^3$, $R^4$, $R^5$, indépendamment les uns des autres, hydrogène, halogène, nitro, alkyle, cyano, alcoxy, alcoxyalcoxy, alkylthioalkylthio, alcoxyalkyle, alkylthioalkyle, alkylthio, trihalogénométhyle ou trihalogénométhoxy.

2. Procédé de préparation de composés selon la revendication 1, caractérisé par le fait que l'on fait réagir un composé phosphore-halogéné (II)

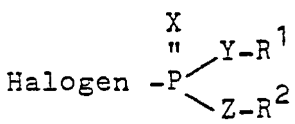

(II)

en présence d'un agent liant les acides avec un phénol correspondant (III)

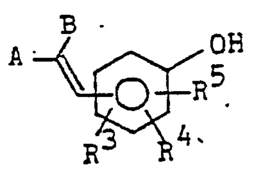

(III)

3. Agents de lutte contre les parasites, contenant un ester d'acide phosphorique selon la revendication 1 et au moins un véhicule solide ou liquide et éventuellement un composé à action synergétique.

4. Utilisation des composés selon la revendication 1, ou des agents selon la revendication 3 pour lutter contre les parasites animaux, en faisant agir les composés ou les agents sur les parasites ou leur biotope.